# EUROPÄISCHE PATENTANMELDUNG

(11) **EP 0 712 621 A1**
(43) Veröffentlichungstag der Anmeldung: **22.05.1996**
(21) Anmeldenummer: 95114609.1
(22) Anmeldetag: 16.09.1995
(51) Int. Cl.: A61K 6/00, A61K 6/09, C09D 4/06, C09J 4/06

(54) **Verfahren zur Erzeugung eines haftfesten, feuchtigkeitsdichten Überzugs aus Kunststoff auf einer Unterlage und dessen Verwendung**

(30) Priorität: 18.11.1994 DE 4441124
(71) Anmelder: Heraeus Kulzer GmbH, 63450 Hanau (DE)
(72) Erfinder: Erdrich, Albert, Dr., D-61231 Bad Nauheim (DE); Fremdt, Sonja, D-35796 Weinbach 2 (DE); Oppawsky, Steffen, D-61350 Bad Homburg (DE)
(74) Vertreter: Grimm, Ekkehard, Dipl.-Phys.

(57) **Zusammenfassung**

Es wird ein Verfahren zur Erzeugung eines haftfesten, feuchtigkeitsdichten Über- zugs aus Kunststoff auf einer Unterlage aus Metall, Keramik, Glas oder Polymer durch Auftragen eines flüssigen Präparats, das Wasser, Lösungsmittel, ein (Meth)Acrylat und ein reaktives Polymer enthält, und Verfestigen dieses Überzuges durch Erwärmen auf eine Temperatur oberhalb 100 °C beschrieben. Ein erstes Präparat wird auf die Unterlage aufgetragen und wenigstens angetrocknet, auf das angetrocknete erste Präparat ein zweites Präparat aufgetragen und wenigstens angetrocknet und danach durch Erhitzen auf eine Temperatur im Bereich 150 bis 400 °C der Überzug verfestigt, wobei das erste Präparat
5 bis 25 Gew% einer wässrigen Copolymer-Suspension, in der das Copolymer aus einer Mischung von 40 - 80 Gew% Acrylnitril und 60 - 20 Gew% Butylacrylat besteht,
5 bis 20 Gew% Wasser
35 bis 75 Gew% polares Lösungsmittel
3 bis 15 Gew% ein bis zu 250 °C nichtflüchtiges (Meth)Acrylat,
mit der Maßgabe, daß die Summe der prozentualen Anteile immer 100% ergibt,
und das zweite Präparat
5 bis 20 Gew% einer 40 - 90 Gew% Lösung eines isocyanatgruppenfreien Polyurethan-Einbrennharzes in Solventnaphta 100
65 bis 85 Gew% Lösungsmittel
3 bis 20 Gew% ein bis zu 250 °C nichtflüchtigen (Meth)Acrylats,
mit der Maßgabe, daß die Summe der prozentualen Anteile immer 100% ergibt,
enthält.

## Beschreibung

Die vorliegende Erfindung betrifft ein Verfahren zur Erzeugung eines haftfesten, feuchtigkeitsdichten Überzugs aus Kunststoff auf einer Unterlage aus Metall, Keramik, Glas oder Polymer durch Auftragen eines flüssigen Präparats, das Wasser, Lösungsmittel, ein (Meth)Acrylat (Methacrylat oder Acrylat) und ein reaktives Polymer enthält, und Verfestigen dieses Überzuges durch Erwärmen auf eine Temperatur oberhalb 100 °C.

Weiterhin betrifft die Erfindung die Verwendung des nach dem Verfahren hergestellten Überzugs.

Ein solches Verfahren ist aus der DE-A1 42 28 530 bekannt. Dort wird eine haftfeste, wasserundurchlässige und hydrolysebeständige Verbundschicht für Metall-, Keramik-, Glas-, Polymer-Kunststoff-Verbunde und eine Dispersion zu deren Herstellung beschrieben. Diese Verbundschicht wird aus einer Phenol-Formaldehyd-Dispersion hergestellt. Hierzu werden zwei Dispersionen vorbereitet und diese beiden Dispersionen zu einem flüssigen Präparat zusammengemischt, das dann auf einer Unterlage aufgebracht wird. Das aufgetragene Präparat wird dann einer 15-minütigen Temperaturbehandlung bei 120 bis 220 °C unterzogen. Die eine Dispersion des Präparats besteht im wesentlichen aus einer Phenol-Formaldehyd-Dispersion, während die zweite Dispersion eine oder mehrere olefinische, ungesättigte, ein- bzw. mehrfunktionelle Methacrylatverbindungen enthält. Es hat sich gezeigt, daß mit diesem Präparat im ausgehärteten Zustand als Haftvermittlerschicht zwischen Metall und Kunststoff relativ hohe Haftwerte erzielt werden können. Ein gewisser Nachteil dieses Präparats bzw. der einzelnen Dispersionen ist jedoch, daß sie nur kurze Lagerstabilitäten zeigen, die nur zwei bis drei Monate betragen und demzufolge für den kommerziellen Einsatz nicht ausreichend sind. Außerdem hängt die Lagerstabilität von den speziellen Lagerbedingungen ab, d.h. um eine nur annähernd annehmbare Lagerstabilitätszeit zu erreichen, müssen die Dispersion bzw. das Präparat gekühlt gelagert werden. Darüberhinaus enthält die eine Dispersion bzw. enthält das Präparat Phenol sowie Formaldehyd, die beide gesundheitsgefährdende Substanzen darstellen. Diese Substanzen zeigen zwar in dem ausgehärteten Präparat eine gewisse Vernetzung, es ist jedoch noch nicht nachgewiesen, daß eine vollständige Vernetzung erfolgt und somit eine Ausdampfung von Formaldehyd völlig ausgeschlossen werden kann.

Ausgehend von dem vorstehend beschriebenen Stand der Technik liegt der vorliegenden Erfindung die Aufgabe zugrunde, ein Verfahren zur Erzeugung eines haftfesten, feuchtigkeitsdichten Überzugs aus Kunststoff zu schaffen, bei dem insbesondere in Bezug auf einen Metall-Kunststoff-Verbund hohe Haftwerte erreicht werden, bei dem darüberhinaus keine gesundheitsgefährdenden Substanzen eingesetzt werden und bei dem die Problematik einer Lagerstabilität nicht gegeben ist.

Die vorstehende Aufgabe wird durch ein Verfahren zur Erzeugung eines haftfesten, feuchtigkeitsdichten Überzugs aus Kunststoff auf einer Unterlage aus Metall, Keramik, Glas oder Polymer durch Auftragen eines flüssigen Präparats, das Wasser, Aceton, ein (Meth)Acrylat und ein reaktives Polymer enthält, und Verfestigen dieses Überzuges durch Erwärmen auf eine Temperatur oberhalb 100 °C gelöst, das dadurch gekennzeichnet ist, daß ein erstes Präparat auf die Unterlage aufgetragen und wenigstens angetrocknet, auf das angetrocknete erste Präparat ein zweites Präparat aufgetragen und wenigstens angetrocknet und danach durch Erhitzen auf eine Temperatur im Bereich 150 bis 400 °C der Überzug verfestigt wird, wobei das erste Präparat
5 bis 25 Gew%
einer wässrigen Copolymer-Suspension, in der das Copolymer aus einer Mischung von 40 - 80 Gew% Acrylnitril und 60 - 20 Gew% Butylacrylat besteht,
5 bis 20 Gew%
Wasser
35 bis 75 Gew%
polares Lösungsmittel
3 bis 15 Gew%
ein bis zu 250 °C nichtflüchtiges (Meth)Acrylat,
mit der Maßgabe, daß die Summe der prozentualen Anteile immer 100% ergibt,
und das zweite Präparat
5 bis 20 Gew%
einer 40 - 90 Gew% Lösung eines isocyanatgruppenfreien Polyurethan-Einbrennharzes in Solventnaphta 100
65 bis 85 Gew% Lösungsmittel
3 bis 20 Gew%
ein bis zu 250 °C nichtflüchtiges (Meth)Acrylat,
mit der Maßgabe, daß die Summe der prozentualen Anteile immer 100% ergibt, enthält.

Mit diesem System aus einem speziellen ersten Präparat und einem speziellen zweiten Präparat wird durch deren aufeinanderfolgende Aufbringung auf einen Träger mit anschließender Temperaturbehandlung ein Überzug geschaffen, der eine dauerhafte, feuchtigkeitsstabile, spaltfreie und hochfeste Verbindung auf einer Unterlage aus Metall, Keramik, Glas oder einem Polymer liefert. Darüberhinaus hat sich gezeigt, daß die beiden eingesetzten Präparate eine hohe Lagerstabilität unter normalen Raumbedingungen zeigen, d.h. es müssen keine besonderen Maßnahmen zur Bevorratung dieser Präparate getroffen werden.

Das Verbundprinzip basiert auf dem schichtweisen Aufbau des Systems und auf dem kontinuierlichen, mechanischen Eigenschaftswechsel von der Unterlage zum Kunststoff oder Kleber hin. Während das direkt aufschmelzende Polyacrylnitril/Butylacrylat-Copolymer eher die hart-zähen Eigenschaften der Unterlage aufweist, ist das vernetzende Polyurethan-Einbrennharz eher zäh-elastisch und überträgt von außen kommende Kräfte unter Verformung teilweise verzehrend nur noch unvollständig in die Grenzschicht zwischen den beiden Präparatschichten. Gleichzeitig werden beide Harzsysteme in sich und miteinander so hoch vernetzt, daß Feuchtigkeit praktisch nicht zu dieser Grenzschicht vordringen kann.

Wesentlich bei dem erfindungsgemäßen Verfahren ist es, daß das System aus den zwei speziellen Präparaten aufgebaut wird, wobei vor der Aufbringung des zweiten Präparats das erste Präparat auf der Unterlage so weit verfestigt ist, daß das zweite Präparat ohne eine wesentliche Vermischung mit dem ersten Präparat auf das erste Präparat aufgebracht werden kann, bevor das gesamte System dann einer Aushärtung unter Temperaturbehandlung im Bereich von 150 bis 400 °C unterworfen wird. Umfangreiche Tests haben gezeigt, daß darüberhinaus an die Verfahrensweise keine hohen Anforderungen bezüglich der Reinheit der Oberfläche der Unterlage gestellt werden müssen. Bekannte Verfahren setzen ein Vorbehandeln der Unterlage voraus, beispielsweise durch chemische Reinigung oder ein Sandstrahlverfahren, um reproduzierbare Ergebnisse zu erreichen. Mit dem erfindungsgemäßen System werden keine wesentlichen Schwankungsbreiten in den Haftfestigkeitswerten beobachtet, falls die Oberfläche der zu beschichtenden Unterlage eine gewisse Verunreinigung, beispielsweise eine fettige Oberfläche, aufweist; zumindest liegen die Haftwerte unabhängig von der Qualität der Oberfläche ausreichend hoch, um die in der Technik, insbesondere im Dentalbereich, geforderten Haftwerte bei Metall-Kunststoff-Verbunden zu erzielen. Ein Reinigungsschritt kann allerdings zusätzlich einen gewissen Vorteil dahingehend bringen, daß sich die Haftwerte erhöhen. Mit dem erfindungsgemäßen Verfahren und den eingesetzten Präparaten konnten Haftwerte bis zu 40 MPa erzielt werden; die Verbundfestigkeit wurde durch thermische Wechselbelastung in Wasser von 5 / 55 °C über 5000 Zyklen nahezu nicht beeinflußt. Ferner zeigten sich mikroskopisch keine Spalte zwischen den Schichten.

Vorzugsweise wird als erstes Präparat ein solches aufgetragen, das eine Suspension eines Acrylnitril/Butylacrylat-Copolymer mit einem Feststoffgehalt von 30 bis 50 Gew% enthält. Wird als Copolymer in der Mischung des ersten Präparats vorzugsweise ein Copolymer aus 60 Gew% Acrylnitril und etwa 40 Gew% Butylacrylat eingesetzt, so werden gute Haftwerte des Kunststoffüberzugs erreicht.

In einer weiteren Modifizierung enthält das zweite Präparat eine etwa 75 Gew%-ige Lösung eines isocyanatgruppenfreien Polyurethan-Einbrennharzes in Solventnaphta 100.

Insbesondere sind hinsichtlich des ersten Präparats an das einzusetzende Lösungsmittel Anforderungen dahingehend zu stellen, daß es sich um ein polares Lösungsmittel handelt. Vorzugsweise wird als ein solches polares Lösungsmittel Ethanol oder Isopropanol eingesetzt; bevorzugt wird allerdings Aceton verwendet, da sich das Aceton relativ schnell verflüchtigt und somit eine gute Vortrocknung des ersten Präparats vor dem Auftragen des zweiten Präparats erreicht werden kann. Die Zeit der Vortrocknung des ersten Präparats bevor das zweite Präparat aufgetragen wird, liegt im Bereich von etwa 1 bis 2 Minuten. Die ausreichende Antrocknung des ersten Präparats vor der Auftragung des zweiten Präparats kann in einfacher Weise durch den Anwender visuell beobachtet werden, da sich die Schicht von dunkel-feucht nach trocken-weißlich verändert.

Bevorzugte Auftragsschichtdicken des ersten und des zweiten Präparats liegen jeweils im Bereich von 1 bis 10 µm, vorzugsweise im Bereich von 4 bis 8 µm. Solche Schichtdicken können in einfacher Weise durch Pinselauftrag erreicht werden. Die Konsistenz des ersten Präparats wird durch Wasser eingestellt derart, daß der Wassergehalt dieses Präparats vorzugsweise im Bereich von 14 bis 18 Gew% liegt. Falls der Wasseranteil zu hoch gewählt wird, wird kein stabiles Präparat erreicht. Während die Vortrocknung des ersten Präparats an der Luft erfolgen kann, was auch für die Vortrocknung des zweiten Präparats gilt, wird die abschließende Aushärtung der aufgetragenen Schichten unter einer Wärmebehandlung durchgeführt. Für großflächige Teile, die mit einem Kunststoffüberzug der beschriebenen Art überzogen werden sollen, hat sich ein Wärmeofen als bevorzugt erwiesen, mit dem die definierte Temperatur zur Aushärtung, die vorzugsweise im Bereich von 200 bis 400 °C liegt, einstellbar ist.

Für die thermische Härtung der aufgetragenen Präparate wurde festgestellt, daß ein wesentliches Kriterium die Sprungtemperatur für die Härtungsreaktion ist. Diese Sprungtemperatur liegt bei etwa 170 °C. Erst wenn der noch nicht verfestigte Überzug für eine gewisse Mindestzeit auf diese Temperatur gebracht ist, setzt eine Härtungsreaktion ein, die zu einer vollständigen Aushärtung und Durchhärtung des Überzugs führt und mit der dann die vorstehend erwähnten Haftwerte erzielbar sind. In einem Ofen, in dem das auszuhärtende Teil eingebracht wird, sollte zur isothermischen Härtung eine Temperatur gewählt werden, die im Bereich von 160 bis 220 °C liegt, bevorzugt im Bereich von 180 bis 200 °C, und zwar sollte sie für 1 bis 10 Minuten, vorzugsweise für 2 bis 4 Minuten, aufrechterhalten werden. In einem Ofen kann diese Temperatur sehr genau eingestellt und über die angegebenen Zeiten beibehalten werden, so daß eine isothermische Erwärmung des Überzugs gewährleistet ist.

Es hat sich gezeigt, daß insbesondere an kleinen Bauteilen, die nach dem Verfahren gemäß der Erfindung mit einem solchen Überzug oder einer entsprechenden Haftschicht, die aus einem solchen Überzug gebildet wird, versehen werden, eine Aushärtung vorteilhaft ist, die mittels eines Heißluftstroms erfolgt. Mit einem Heißluftstrom kann Wärme definiert auf bestimmte Bereiche eines kleinen, auch komplex gestalteten Bauteils aufgebracht werden, wobei in einem solchen Fall vorzugsweise die Temperatur des Heißluftstroms auf 300 bis 350 °C eingestellt und für etwa 10 bis 25 Sekunden appliziert wird, oder mit einer Temperatur von 250 bis 300 °C für 20 bis 40 Sekunden oder mit einer Temperatur von 200 bis 250 °C für 30 bis 60 Sekunden. Mit diesen erhöhten Temperaturen wird die Sprungtemperatur von etwa 170 °C in einer sehr kurzen Zeit überschritten und die Aushärtreaktion eingeleitet. Es hat sich gezeigt, daß gerade ein Heißluftstrom zu einer gleichmäßigen Erwärmung eines Bauteils auch im Bereich von Hinterschneidungen oder Vorsprüngen führt, da dieser Heißluftstrom gleichmäßig um das Bauteil herumführbar ist. Es hat sich darüberhinaus gezeigt, daß eine ausreichende Aushärtung bzw. das Ende der Härtung unter Einsatz eines Heißluftstroms auch visuell beobachtet werden kann. Es tritt nämlich gegen Ende der Aushärtung eine Umfärbung des Überzugs von farblos-glänzend nach mittelbraun-matt ein. Eine dunkelbraun-schwarze Verfärbung zeigt eine Überhitzung der Schicht an, die dann für Verbundzwecke unbrauchbar ist. Auf diese Weise können auch Bauteile flächenbereichsweise dadurch ausgehärtet werden, daß die Heißluft von Flächenbereich zu Flächenbereich geführt wird, wobei eine Verschiebung erst dann erfolgt, wenn die erforderliche matt-mittelbraune Umfärbung, die die ausreichende Aushärtung signalisiert, eingetreten ist.

Wie schon erwähnt, hat sich gezeigt, daß auch hinsichtlich des Auftragens der einzelnen Präparate die ausreichende Vortrocknung bzw. Vorhärtung visuell beobachtet werden kann. Das erste Präparat zeigt nach seinem Auftragen ein sichtbar nasses Erscheinungsbild, das nach dem Antrocknen zu einer leichten Weißverfärbung übergeht. Nach dieser Weißverfärbung ist das erste Präparat ausreichend vorbereitet, um das zweite Präparat aufzutragen. Auch das zweite Präparat zeigt wiederum nach seinem Auftrag zunächst ein naß-glänzendes Erscheinungsbild, das nach einer geringen Antrocknungszeit zu einem seidenmatten Erscheinungsbild übergeht. Nach Erreichen dieses Zustands des zweiten Präparats kann die abschließende Aushärtung unter Wärmeeinwirkung erfolgen.

Ein weiterer Vorteil, der sich mit dem erfindungsgemäßen Verfahren und den dabei eingesetzten Präparaten ergibt, ist derjenige, daß nach Abkühlen des ausgehärteten Überzugs dieser für die Copolymerisation mit (Meth)Acrylat-Klebern, -Kunststoffen oder -Komposits vorbereitet ist und auf unbestimmte Zeit aktiv verbleibt, sofern er nicht verschmutzt wird. Dieser aktive Zustand bedeutet, daß auf diesen Überzug Kunststoffe oder Komposits mit reproduzierbaren Haftergebnissen aufgebracht werden können. Lagerversuche derart vorbereiteter, mit einem Überzug versehenen Unterlagen unter Raumbedingungen zeigten sogar nach einem Monat keine Veränderung der Verbundfestigkeit im Vergleich mit einem unmittelbar nach der Aufbringung des Überzugs mit Kunststoff beschichteten Probekörpers.

Außer auf Metallträgern als Unterlage für den Überzug konnte das erfindungsgemäße Beschichtungsverfahren erfolgreich für dentale und technische Keramiken, sowohl auf Feldspatbasis als auch auf Aluminiumoxidbasis, sowie auf Duroplasten mit Temperaturfestigkeiten größer 180 °C, angewandt bzw. bei ihnen durchgeführt werden. Die mittels des Verfahrens erzeugten Überzüge unter Einsatz der angegebenen beiden Präparate stellt eine optimale Haftvermittlerschicht für einen (Meth)Acrylat-Klebstoff zum Verbinden von zwei mit dem Überzug jeweils versehenen Unterlagen dar.

Weitere vorteilhafte Merkmale des Verfahrens ergeben sich aus den weiteren Unteransprüchen.

Nachfolgend werden verschiedene Ausführungsbeispiele und Versuchsergebnisse detailliert erläutert.

Ein erstes Beispiel zur Herstellung eines Überzugs auf Metall wurde wie folgt durchgeführt:
1. Durch Sandstrahlen wird die Metall-Unterlage mechanisch gereinigt und ihre Oberfläche vergrößert. Es wird bevorzugt Aluminiumoxid (Korund) eingesetzt. Die Körnung kann 50 bis 250 µm betragen, bevorzugt wird 100 bis 150 µm. Der Strahldruck kann 2 bis 5 bar betragen, bevorzugt 2 bis 3 bar, primär bedingt durch die Empfindlichkeit zahntechnischer Arbeiten, mit wenig Einfluß auf das Verbundergebnis.
2. Die Oberfläche wird mit einem harten Pinsel ohne weitere Hilfsmittel grob entstaubt.
3. Präparat I wird aufgetragen: wässrig-acetonische Dispersion aus einem Acrylnitril/Butylacrylat-Copolymer und einem bis zu 250 °C nichtflüchtigem (Meth)Acrylat, bevorzugt das Urethanmethacrylat aus Trimethylhexyldiisocyanat und Hydroxyethyl(meth)acrylat (UEDMA).
   - Beispiel Rezeptur 1:: 9 g Copolymer-Suspension
   10 g Aqua dest.
   40 g Aceton p.A.
   7 g UEDMA
   - Beispiel Rezeptur 2:: 12 g Copolymer-Suspension
   10 g Aqua dest.
   40 g Aceton p.A.
   8 g BisGMA (2,2-Bis-[p-(2-hydroxy-2-methacryloyloxpropoxy)-phenyl]propan)
   - Beispiel Rezeptur 3:: 6 g Copolymer-Suspension
   10 g Aqua dest.
   40 g Aceton p.A.
   3 g PETA (Pentaerythritol-tetra-acrylat)
   Das Präparat I wird mit einem Pinsel satt auf dem Metallkörper aufgetragen und man läßt es bei Raumbedingungen 2 bis 3 Minuten antrocknen, bis eine leicht weiße Verfärbung an der Oberfläche eintritt.
4. Präparat II wird aufgetragen: Acetonische Lösung aus einem vernetzenden Polyurethan-Einbrennharz und einem bis zu 250 °C nichtflüchtigen (Meth)Acrylat, bevorzugt das Urethandimethacrylat (UEDMA) aus Trimethaylhexyldiisocyanat und Hydroxyethyl(meth)acrylat.
   - Beispiel Rezeptur 4:: 5 g Urethan-Einbrennharz-Lösung
   2,5 g UEDMA
   25 g Aceton p.A.
   - Beispiel Rezeptur 5:: 2,5 g Urethan-Einbrennharz-Lösung
   5 g BisGMA
   25 g Aceton p.A.
   - Beispiel Rezeptur 6:: 7,5 g Urethan-Einbrennharz-Lösung
   1,5 g PETA
   25 g Aceton p.A.
   Das Präparat II wird mit einem Pinsel satt aufgetragen und man läßt es bei Raumbedingungen 2 bis 3 Minuten antrocknen, bis sich eine farblose, seidenmatte Oberfläche einstellt.
5. Die thermische Härtung der aufgetragenen Schichten wird dann bei einer Temperatur oberhalb 170 °C durchgeführt.
6. Nach dem Abkühlen der ausgehärteten Schichten sind diese für die Copolymerisation mit (Meth)Acrylat-Klebern, -Kunststoffen oder -Komposits vorbereitet.
7. Als Verbundprüfungsmaterialien wurden handelsübliche Füllungs- und Verblendkomposits sowie Kleber der Firma Heraeus Kulzer GmbH verwendet, die aus anorganischen Füllstoffen und di- bis polyfunktionellen (Meth)Acrylatmatrices aufgebaut waren. Die Verbundprüfung wurde gemäß einem Normvorschlag zur ISO 10 477, geltend für zahntechnische Kronen- und Brücken-Verblendwerkstoffe, durchgeführt. Dabei wurden die nachfolgenden Ergebnisse erzielt.

Auf eine einer Verblendeinheit entsprechenden Fläche (ca. 100 mm² = etwa 8 mm x 12 mm) wird im Mittel etwa so viel an Präparat I aufgebracht, daß nach dem Antrocknen 10 ± 1 mg Substanz zurückbleiben und eine Schichtstärke von 6 ± 1 µm erzeugt wird. Das Präparat II wird ebenso stark aufgebracht, so daß die ungehärtete Gesamtschichtstärke des Überzugs 12 ± 1 µm beträgt. Durch die thermische Härtung mit Heißluft (siehe Punkt 5.) bleibt eine Menge von 11 ± 1 mg und eine Schichtdicke von 7 ± 1 µm zurück. Nach Verbindung dieser Schicht mit dem Kunststoff, Kleber oder Komposit werden im Scherversuch folgende Verbundfestigkeiten erzielt:

| Verbundfestigkeit im Scherversuch Ergebnisbereich für hochgoldhaltige und goldreduzierte Guß- und Keramikaufbrennlegierungen sowie Nichtedelmetallegierungen | nach Kochtest in Wasser 30 min/100°C MPa | nach Thermowechsellast 5000 Zyklen in Wasser: 30 s/5 °C - 10 s/RT -30 s/55°C - 10 s/RT MPa |
|---|---|---|
| 1. Behandlung nach Punkt 1, 2, 3, 4, 5, 6, 7: DC-Op - DC-Vbm | 18 - 23 | 17 - 22 |
| 2. Behandlung nach Punkt 1, 2, 4, 3, 5, 6, 7: DC-Op - DC-Vbm | 5 - 10 | 3 - 8 |
| 3. Behandlung nach Punkt 1, 2, 3, 4, 5, 6, 7: (ARTG-Op) Zahnfüllungsmaterial CHAR | 30 - 35 | 27 - 32 |
| 4. Behandlung nach Punkt 1, 2, 3, 4, 5, 6, 7: ARTG-Op ARTG | 33 - 40 | 30 - 35 |
| 5. Behandlung nach Punkt 1, 2, 3, 4, 5: 300 °C Heißluft (HL)-20 s, 6, 7: ARTG-Op - DC-Vbm | 20 - 25 | 19 - 24 |
| Erläuterungen: DC-Op : zahnfarbener, lichthärtbarer Zweikomponenten-Metallabdecklack auf Basis von Methylmethacrylat, 2,2-Bis-[p-(2-hydroxy-2-methacryloyloxypropoxy)-phenyl]propan (BisGMA), Titandioxid und farbgebenden Pigmenten. DC-Vbm: zahnfarbenes, lichthärtbares Verblendmaterial für dentale Metallgerüste auf Basis von Urethanethylenglykol-dimethacrylat (UEDMA), Dodekandiol-dimethacrylat (DoDDMA), Siliciumdioxid und gemahlenem Vorpolymerisat. ART-Op: zahnfarbener, lichthärtbarer Einkomponenten-Metallabdecklack auf Basis von UEDMA, BisGMA, Pentaerythritol-tetracrylat (Penta), Siliciumdioxid, Titandioxid und farbgebenden Pigmenten. ARTG: lichthärtbares Polymerglas für dentale Anwendung auf Basis von feinstgemahlenem Barium-Aluminium-Silikatglas, rheologisch wirksamer Kieselsäure und einem Gemisch bi- und mehrfunktioneller (Meth)Acrylsäureestern. | | |

Die Tabelle zeigt, daß unter Einhaltung der Behandlungsschritte, wie sie vorstehend erläutert sind, sehr hohe Verbundfestigkeiten im Scherversuch erreicht werden konnten. Die Tests im Rahmen von Thermowechselbelastungen zeigen darüberhinaus, daß auch ein dauerhafter, feuchtestabiler Verbund gegeben ist.

In der vorstehenden Zusammenstellung der Testergebnisse ist insbesondere auf den Test 2. hinzuweisen, bei dem ein Versuch vorgenommen wurde, in dem die Verfahrensschritte 3 und 4 ausgetauscht wurden, d.h. es wurde zunächst das Präparat II aufgetragen und angetrocknet, bevor das Präparat I dann aufgetragen wurde. Dieser Austausch der Aufbringungsfolge der Präparate I und II führt zu einem starken Abfall der Haftwerte, wodurch belegt wird, daß unbedingt die Auftragung in der vorstehend erläuterten und erfindungsgemäßen Reihenfolge der Präparate I und II erfolgen soll.

Mit weiteren Metallen nicht-dentaler Art und Verwendung wurden nach Optimierung und Anpassung der thermischen Härtungsparameter auf die Wärmekapazität der Verbundfläche vergleichbare Ergebnisse im Verbund zu (Meth)Acrylat-Kunststoffen, -Komposits oder -Klebern erreicht. Auch bei der Verklebung von zwei Metallen mit entsprechenden (Meth)Acrylat-Klebern konnten vergleichbare, feuchtebeständige Verbundfestigkeiten erzielt werden.

## Patentansprüche

1. Verfahren zur Erzeugung eines haftfesten, feuchtigkeitsdichten Überzugs aus Kunststoff auf einer Unterlage aus Metall, Keramik, Glas oder Polymer durch Auftragen eines flüssigen Präparats, das Wasser, Lösungsmittel, ein (Meth)Acrylat und ein reaktives Polymer enthält, und Verfestigen dieses Überzuges durch Erwärmen auf eine Temperatur oberhalb 100 °C, dadurch gekennzeichnet, daß ein erstes Präparat auf die Unterlage aufgetragen und wenigstens angetrocknet, auf das angetrocknete erste Präparat ein zweites Präparat aufgetragen und wenigstens angetrocknet und danach durch Erhitzen auf eine Temperatur im Bereich 150 bis 400 °C der Überzug verfestigt wird, wobei das erste Präparat
5 bis 25 Gew% einer wässrigen Copolymer-Suspension, in der das Copolymer aus einer Mischung von 40 - 80 Gew% Acrylnitril und 60 - 20 Gew% Butylacrylat besteht,
5 bis 20 Gew% Wasser
35 bis 75 Gew% polares Lösungsmittel
3 bis 15 Gew% ein bis zu 250 °C nichtflüchtiges (Meth)Acrylat,
mit der Maßgabe, daß die Summe der prozentualen Anteile immer 100% ergibt,
und das zweite Präparat
5 bis 20 Gew% einer 40 - 90 Gew% Lösung eines isocyanatgruppenfreien Polyurethan-Einbrennharzes in Solventnaphta 100
65 bis 85 Gew% Lösungsmittel
3 bis 20 Gew% ein bis zu 250 °C nichtflüchtigen (Meth)Acrylats,
mit der Maßgabe, daß die Summe der prozentualen Anteile immer 100% ergibt,
enthält.

2. Verfahren nach Anspruch 1, dadurch gekennzeichnet, daß als erstes Präparat ein solches aufgetragen wird, das eine wässrige Suspension aus Acrylnitril/Butylacrylat-Copolymer mit einem Feststoffgehalt von 30 bis 50 Gew% enthält.

3. Verfahren nach Anspruch 2, dadurch gekennzeichnet, daß das Copolymer der Suspension des ersten Präparats aus einem Gemisch von etwa 60 Gew% Acrylnitril und etwa 40 Gew% Butylacrylat besteht.

4. Verfahren nach einem der Ansprüche 1 bis 3, dadurch gekennzeichnet, daß als Lösung für das zweite Präparat eine etwa 75 Gew%-ige Lösung eines isocyanatgruppenfreien Polyurethan-Einbrennharzes in Solventnaphta 100 eingesetzt wird.

5. Verfahren nach einem der vorhergehenden Ansprüche, dadurch gekennzeichnet, daß in dem ersten Präparat und/oder dem zweiten Präparat als Lösungsmittel Ethanol oder Isopropanol, vorzugsweise Aceton, eingesetzt werden.

6. Verfahren nach einem der Ansprüche 1 bis 5, dadurch gekennzeichnet, daß das erste Präparat, das aufgetragen wird,
12 bis 18 Gew% der wässrigen Copolymer-Suspension,
14 bis 18 Gew% Wasser,
50 bis 60 Gew% Aceton und
7 bis 12 Gew% (Meth)Acrylat enthält,
mit der Maßgabe, daß die Summe der prozentualen Anteile immer 100% ergibt.

7. Verfahren nach einem der Ansprüche 1 bis 6, dadurch gekennzeichnet, daß das zweite Präparat, das aufgetragen wird,
8 bis 15 Gew% Polyurethan in Solventnaphta 100,
70 bis 80 Gew% Aceton und
5 bis 12 Gew% (Meth)Acrylat enthält,
mit der Maßgabe, daß die Summe der prozentualen Anteile immer 100% ergibt.

8. Verfahren nach einem der Ansprüche 1 bis 7, dadurch gekennzeichnet, daß das erste und das zweite Präparat jeweils in einer Schichtdicke im Bereich von 1 bis 10 µm, vorzugsweise von 4 bis 8 µm, aufgetragen werden.

9. Verfahren nach einem oder mehreren der Ansprüche 1 bis 8, dadurch gekennzeichnet, daß auf den Überzug ein Heißluftstrom zur Verfestigung gerichtet wird.

10. Verfahren nach Anspruch 9, dadurch gekennzeichnet, daß ein Heißluftstrom mit einer Temperatur im Bereich von 200 bis 400 °C auf den Überzug gerichtet wird.

11. Verfahren nach einem der Ansprüche 1 bis 10, dadurch gekennzeichnet, daß das erste Präparat auf ein Dentalprothesenteil, wie Brücke, Krone oder Prothesensattel, aufgetragen wird.

12. Verfahren nach Anspruch 11, dadurch gekennzeichnet, daß ein Dentalprothesenteil aus einer edelmetallhaltigen Legierung oder einer Nichtedelmetall-Legierung mit dem Überzug versehen wird.

13. Verfahren nach einem der Ansprüche 1 bis 12, dadurch gekennzeichnet, daß die Unterlage mechanisch oder chemisch vor dem Auftragen des ersten Präparats gereinigt wird.

14. Verfahren nach Anspruch 1, dadurch gekennzeichnet, daß das erste Präparat, das aufgetragen wird, als (Meth)Acrylat ein Urethanmethacrylat (UEDMA), 2,2-Bis-[p-(2-hydroxy-2-methacryloyloxpropoxy)-phenyl]propan (BisGMA) oder Pentaerythritol-tetra-acrylat (PETA) enthält.

15. Verfahren nach Anspruch 1, dadurch gekennzeichnet, daß das zweite Präparat, das aufgetragen wird, als (Meth)Acrylat ein Urethanmethacrylat (UEDMA), 2,2-Bis-[p-(2-hydroxy-2-methacryloyloxpropoxy)-phenyl]propan (BisGMA) oder Pentaerythritol-tetra-acrylat (PETA) enthält.

16. Verfahren nach den Ansprüchen 1 oder 15, dadurch gekennzeichnet, daß das zweite Präparat, das aufgetragen wird, ein Polyurethan-Einbrennharz auf Basis eines monomerenarmen Hexamethylendi-isocyanat-Polymerisats und eines Oxims enthält.

17. Verfahren nach Anspruch 16, dadurch gekennzeichnet, daß als Oxim Butanonoxim oder Caprolactam verwendet wird.

18. Vefahren nach einem der Ansprüche 1 oder 15, dadurch gekennzeichnet, daß ein Urethanmethacrylat (UEDMA), das aus Trimethylhexyldiisocyanat und Hydroxyethyl(meth)acrylat gebildet ist, verwendet wird.

19. Verwendung des nach einem der Ansprüche 1 bis 18 hergestellten Überzugs als Haftvermittlerschicht für einen (Meth)Acrylat-Klebstoff zum Verbinden von zwei jeweils mit dem Überzug versehenen Unterlagen miteinander.
